# EUROPÄISCHE PATENTANMELDUNG

(11) **EP 0 639 352 A1**
(43) Veröffentlichungstag der Anmeldung: **22.02.1995**
(21) Anmeldenummer: 94113002.3
(22) Anmeldetag: 19.08.1994
(51) Int. Cl.: A61B 17/66

(54) **Vorrichtung zum Behandeln von Knochen**

(30) Priorität: 20.08.1993 DE 4328015
(71) Anmelder: Alumedica Aluminium-Bearbeitung Medizintechnik GmbH, D-78187 Geisingen (DE); Treu Instrumente GmbH, D-78532 Tuttlingen (DE)
(72) Erfinder: Bittenbinder, Marc, D-78532 Tuttlingen (DE); Treu, Ernst, D-78532 Tuttlingen (DE); Henkemeyer, Herbert Dr., D-78052 Pfaffenweiler (DE)
(74) Vertreter: Hiebsch, Gerhard F., Dipl.-Ing.

(57) **Zusammenfassung**

Bei einer Vorrichtung zum Behandeln eines Knochens mit einem in diesen einsetzbaren Nagel (12), dessen Mittelachse Durchbrüche zur Aufnahme von Schrauben, Bolzen od. dgl. Stiftelementen (22,23,23p) queren und der an einem Ende zumindest ein Rundloch (18), insbesondere ein Paar von Rundlöchern aufweist, ist im anderen Endbereich des Nagels (12) ein Längsschlitz (20) zur Aufname wenigstens einer darin gleitbaren Schraube eines Bolzens od. dgl. Stiftelements (23,23p) angeordnet. Diese/s ist mit der/den Schraube/n od. dgl. Stiftelement/en (22) des anderen Nagelendes (16) durch eine längenveränderbare Verstelleinrichtung (28) verbunden.

## Beschreibung

Die Erfindung betrifft eine Vorrichtung zum Behandeln eines Knochens mit einem in diesen einsetzbaren Nagel, dessen Mittelachse Durchbrüche zur Aufnahme von Schrauben, Bolzen od.dgl. Stiftelementen queren, wobei an einem Ende des Nagels zumindest ein Rundloch, insbesondere ein Paar von Rundlöchern, als Durchbruch vorgesehen ist.

Derartige Marknägel mit einer endwärtigen Spitze werden in der Operationstechnik zur Nagelung von Knochenbrüchen eingesetzt. Beispielsweise setzt der Operateur bei einem Femurbruch in der Schaftachse eine Ahle an oder einen Führungsdraht in der Mitte der Fossa trochantrica. Eine Kugelspitze des Führungsdrahtes wird bis zur Frakturstelle eingeschoben, dann wird bei einer der Operationsmethoden der so entstandene Kanal zur Einführung des Marknagels aufgefräst und letzterer eingeschlagen. Anschließend erfolgt eine distale Verriegelung mittels wenigstens einer den Marknagel durchsetzenden Schraube, deren beide Enden im Knochen festliegen. Selbstverständlich kann ein Nagel auch ohne Auffräsung eingebracht werden.

Hierzu werden bevorzugt sog. Verriegelungsschrauben eingesetzt, beispielsweise bei einer Tibia zumindest zwei Schrauben.

Zur Extraktion des Nagels werden die Schrauben entfernt, ein Ausschlägeradapter auf das proximale Ende des Marknagels geschraubt und letzterer mit einem Schlitzhammer ausgetrieben.

Marknägel sind entweder -- bevorzugt anterior -- der Länge nach mit einem Schlitz versehen oder weisen einen geschlossenen Querschnitt von beispielsweise 8 mm bis 16 mm Durchmesser auf, die Verriegelungsschrauben haben ein Teilgewinde mit einem Außendurchmesser von etwa 4,5 mm.

In Kenntnis dieses Standes der Technik hat sich der Erfinder das Ziel gesetzt, eine Vorrichtung der eingangs erwähnten Art so auszugestalten, daß sie für die Verlängerung von Knochen eingesetzt zu werden vermag.

Zur Lösung dieser Aufgabe führt die Lehre des Patentanspruches 1; im anderen Endbereich des Nagels ist ein Längsschlitz zur Aufnahme wenigstens eines darin gleitbaren Bolzens, einer Schraube od.dgl. Stiftelement angeordnet, und letzteres wird mit der/den Schraube/n od.dgl. Stiftelement/en des anderen Nagelendes durch eine längenveränderbare Verstelleinrichtung verbunden. Diese Maßgabe hat zur Folge, daß die den Längsschlitz durchsetzenden Schrauben in diesem geführt werden; da sie zudem den Knochen bzw. die Knochenrinde -- in dieser fixiert -- durchgreifen, kann der entsprechende Teil des Knochens durch die Spreizwirkung der Verstelleinrichtung nach Bedarf verschoben werden.

Im Rahmen der Erfindung liegt jedoch auch eine andere Lösung nach dem zweiten unabhängigen Patentanspruch; der Nagel ist an seinen beiden Enden jeweils mit wenigstens einem Rundloch -- bevorzugt mit einem Paar von Rundlöchern -- ausgestattet, in welche jeweils eine Schraube od.dgl. Stiftelement für die erwähnte Verstelleinrichtung angeschlossen werden kann, zugleich ist der Nagel zwischen den beiden Endbereichen längenveränderlich ausgebildet, bevorzugt als Teleskop.

Bei einer besonderen Ausführung kann auch der mit dem Längsschlitz versehene Nagel in sich längenveränderlich ausgebildet sein, so daß die Wirkungsweise beider oben beschriebener Ausführungsformen kombiniert zu werden vermag.

In all diesen Applikatlonsfällen ist die teleskopartig einstellbare Verstelleinrichtung in einem Abstand parallel zum Nagel vorgesehen, da sie außerhalb des zu behandelnden Knochens angeordnet sein muß.

Weitere besondere Ausgestaltungen des Erfindungsgegenstandes sind den abhängigen Patentansprüchen zu entnehmen.

So ist zumindest ein Rundloch für ein Stiftelement -- bevorzugt ein Paar von Rundlöchern -- im proximalen Endbereich des Nagels angeordnet und der Längsschlitz im distalen Bereich, in welchem der Nagel in an sich bekannter Weise vorteilhafterweise mit einer Spitze versehen ist.

Auch kann dem Längsschlitz zumindest ein Rundloch zugeordnet sein -- bevorzugt ein Paar von Rundlöchern --, wobei in einer Ausführung dieses Rundloch seitlich des Längsschlitzes liegt, bei einer anderen Ausführung in der vom Längsschlitz bestimmten Geraden, also bevorzugt in der Nagelmittelachse.

Nach einem weiteren Merkmal der Erfindung ist zwischen den beiden gegeneinander bewegbaren Abschnitten des längenveränderlichen Nagels eine Rücklaufbremse vorgesehen.

Erfindungsgemäß kann zumindest ein Abschnitt ein Hohlprofil und in diesem ein Stab des anderen Abschnittes kolbenartig verschiebbar sein. Jedoch ist es auch möglich, daß beide Abschnitte Hohlprofile mit in ihnen relativ bewegbarem Stab sind. In diesem Falle umfaßt die erwähnte Rücklaufbremse bevorzugt eine Zahnleiste an einem Bewegungspartner, mit der ein Anschlagelement des anderen Gleitpartners so kämmt, daß eine Hemmung gegen die Hubrichtung des Stabes entsteht. Das Rast- oder Anschlagorgan wird bevorzugt als Federzunge ausgebildet, deren freies Ende gegen eine Stufenfläche der erwähnten Zahnleiste anschlägt.

Dank dieser Vorrichtung kann durch das Eingreifen der Gewindeflanken der erwähnten Schanz'schen Schrauben im Nagel und der Cortikalis eine gleichmäßige Kraftverteilung erzielt werden. Beim Erreichen der vorgegebenen Maximallänge werden die Schanz'schen Schrauben am proximalen Ende gegen Verriegelungsschrauben ausgestauscht. Am distalen Ende erfolgt das Herausnehmen der Schanz'schen Schrauben nach dem Einbringen der Verriegelungsschrauben in besondere Bohrungen. Die distalen Schanz'schen Schrauben werden an der proximalen Seite des Langloches oder Längsschlitzes eingebracht und mit der Distrahierung der Verstelleinrichtung in distaler Richtung verschoben.

Nach dem Erreichen der maximal gewünschten Verlängerung wird die Verriegelungsschraube im distalen Bereich in ein Bohrloch eingesetzt, die Schanz'schen Schrauben entfernt und dann noch proximal die Schanz'schen Schrauben durch Verrlegelungsschrauben ersetzt.

Die soeben beschriebene Vorgehensweise ist für den nicht verlängerbaren Nagel gedacht; bei dem längenveränderlichen Nagel nach dem zweiten unabhängigen Patentanspruch werden die Schanz'schen Schrauben im Marknagel und in der Cortikalis verankert; der Marknagel bleibt proximal und distal gleichermaßen fixiert. In diesem Falle wird die Belastung von der Verstelleinrichtung bzw. dem Fixateur und den Schanz'schen Schrauben in starrer Verbindung mit dem Marknagel getragen, ohne daß auf den Fixateur Biegekräfte einwirken würden. Beim Erreichen der maximal gewünschten Länge werden die Schanz'schen Schrauben durch Verriegelungsschrauben ersetzt.

Durch die Verbindung der Schanz'schen Schrauben mit dem Marknagel treten hier wesentlich geringere Biegekräfte bezüglich der Verstelleinrichtung auf, ohne daß sich die auf die Bohrungen oder Rundlöcher auswirkenden Kräfte durch die Cortikalis wesentlich verändern würden.

Der eigentliche Verlängerungsvorgang wird durch manuelle, mechanische Krafteinflüsse über die Verstelleinrichtung von außen her bewirkt. Im Rahmen der Erfindung können die Kräfte auch auf elektrischem, hydraulischem oder pneumatischem Wege erfolgen.

Ist die gewünschte Verlängerung erfolgt, können auch hier die Schanz'schen Schrauben durch Verriegelungsschrauben ersetzt werden.

Weitere Vorteile, Merkmale und Einzelheiten der Erfindung ergeben sich aus der nachfolgenden Beschreibung bevorzugter Ausführungsbeispiele sowie anhand der Zeichnung; diese zeigt in
- Fig. 1:: eine Seitenansicht einer Vorrichtung zur axialen Dehnung eines Unterschenkelknochens mit einem intraossären Nagel;
- Fig. 2:: die Vorrichtung nach Fig. 1 in anderer Betriebsstellung;
- Fig. 3, 4:: unterschiedliche Details weiterer Ausführungen der Vorrichtung;
- Fig. 5, 6:: den Fig. 1, 2 entsprechende Wiedergaben einer anderen erfindungsgemäßen Vorrichtung mit längenveränderlichem Nagel;
- Fig. 7:: die Seitenansicht eines vergrößerten Abschnitts eines längenveränderlichen Nagels;
- Fig. 8:: ein teilweise geschnittener längenveränderlicher Nagel;
- Fig. 9:: den vergrößerten Querschnitt durch den Nagel, geschnitten gemäß Linie IX-IX in Fig. 10;
- Fig. 10:: den vergrößerten Ausschnitt aus Fig. 8 nach deren Feld X;
- Fig. 11,12:: den Fig. 1, 2 entsprechende schematisierte Darstellungen einer Vorrichtung zur Dehnung eines Oberschenkelknochens oder Femur.

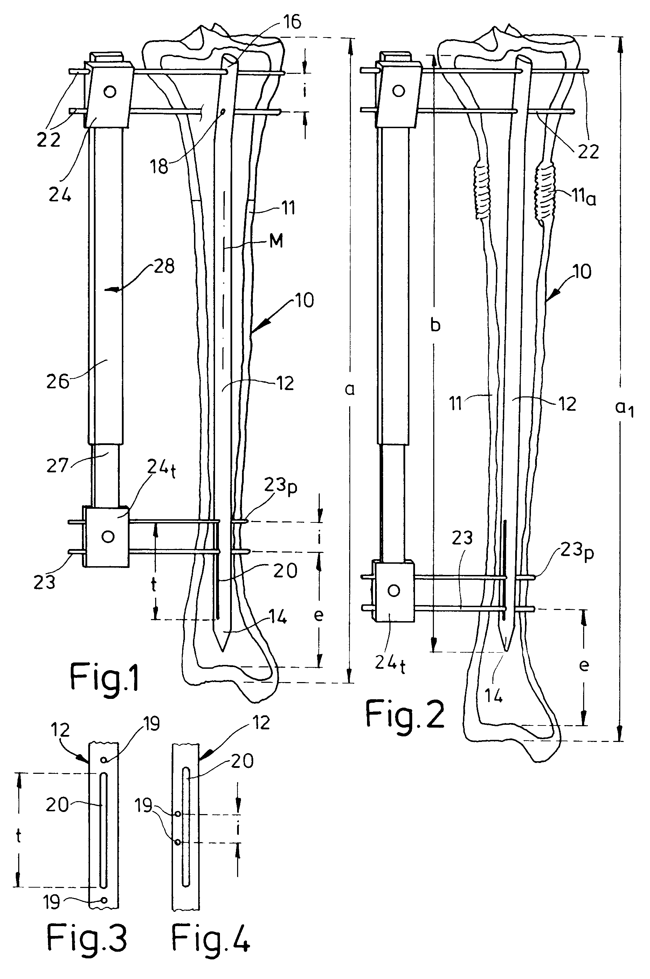

In einem durch die Kontur 10 angedeuteten Unterschenkelknochen der Länge a verläuft ein metallischer Nagel 12 konstanter Länge b, welcher mit seiner Nagelspitze 14 am rumpfnahen Knochenende angesetzt und in dessen Mark eingeschlagen worden ist. Das leicht abgewinkelte proximale Ende 16 des Marknagels 12 durchsetzen in einem Abstand i zueinander zwei Querbohrungen 18, und in der distalen Nagelspitze 14 verläuft in der Mittelachse M des Marknagels 12 ein Langloch oder Längsschlitz 20 einer Länge t von beispielsweise 40 bis 80 mm. Gemäß Fig. 3, 4 können dem Längsschlitz 20 axial oder radial weitere Querbohrungen 19 zugeordnet sein.

Die beiden proximalen Querbohrungen 18 nehmen jeweils eine sie und die Tibia 10 durchsetzende Schraube 22 auf, die -- in der Zeichnung links -- außerhalb der Tibia 10 in einem Haltekopf 24 eines Zylinderteiles 26 sitzt. In diesem lagert teleskopartig verschieblich ein Kolbenprofil 27, das einen Haltekopf 24t für zwei andere Schrauben 23 trägt sowie mit dem Zylinderteil 26 eine/n längenveränderliche/n Verstelleinrichtung bzw. Fixateur 28 bildet. Die Schrauben 22, 23 stehen zueinander parallel.

Die in der Zeichnung unteren Schrauben 23,23p durchgreifen die Tibia 10 sowie den Längsschlitz 20, wobei die hier proximale Schraube 23p in Fig. 1 dem oberen Schlitzende innenseitig anliegt.

Die Nagelspitze 14 dient zur Führung der -- durch Erhöhung der wirksamen Länge des Fixateurs 28 -- im Längsschlitz 20 wandernden Schrauben 23, 23p; der Abstand e der unteren Schraube 23 zum distalen Knochenende bleibt dabei unverändert. Dank dieser Lagerung der Gewindeflanken der Schrauben 23, 23p im Marknagel 12 sowie in der Knochenrinde 11 -- deren Verlängerungsbereich in Fig. 2 mit 11a bezeichnet ist -- entsteht eine gleichmäßige Kraftverteilung.

Ist die maximale Länge a1 der Tibia 10 erreicht, werden die auch als Schanz'sche Schrauben bezeichneten Schrauben 22 durch -- nicht dargestellte -- sog. Verriegelungsschrauben ersetzt, welche den Marknagel 12 im Knochen verankert halten. Die Verankerung des distalen Nagelbereiches -- Nagelspitze 14 -- erfolgt durch Verriegelungsschrauben, welche die Bohrungen 19 durchsetzen. Dann werden die Schanz'schen Schrauben 23, 23p ebenfalls der Tibia 10 entnommen.

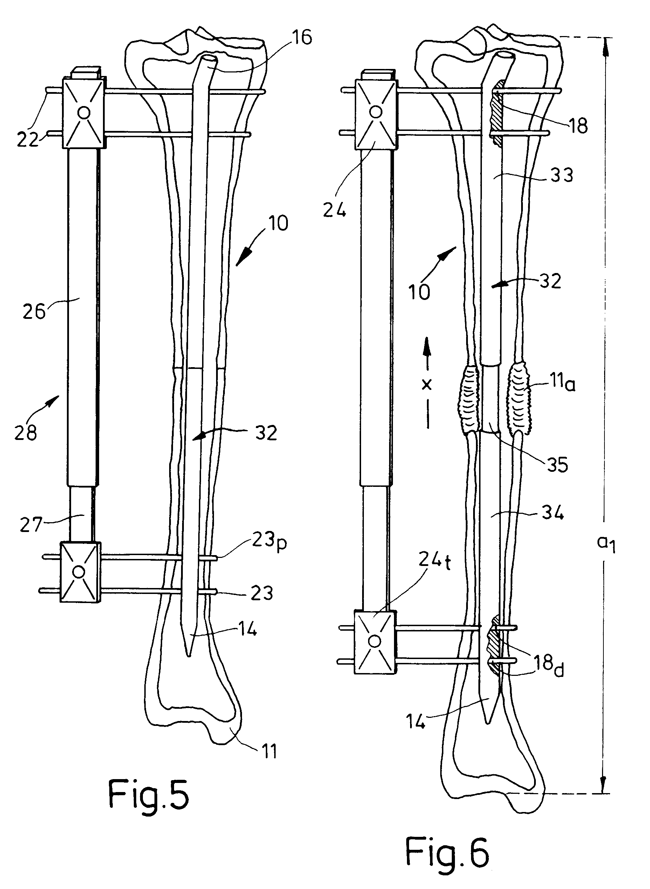

Die Fig. 5 und 6 zeigen einen Verlängerungsnagel 32, der seinerseits längenveränderlich ausgebildet ist. Er ist etwa in Längsmitte unterteilt, und die beiden so entstehenden Nagelteile 33, 34 sind zur Aufnahme eines sie verbindenden Axialstücks 35 teilweise hohl. Dies ermöglicht die teleskopartige Verlängerung des Verlängerungsnagels 33 beim Auseinanderziehen der beiden Nagelteile 33, 34. Bei dieser Ausführung sind sowohl im proximalen Nagelteil 33 als auch im Bereich der distalen Nagelspitze 14 Querbohrungen 18, 18d für die Schanz'schen Schrauben 22, 23, 23p vorgesehen, d.h. der Verlängerungsnagel 32 bleibt durch diese proximal und distal fixiert.

Hier wird die Belastung vom Fixateur 28 und den Schanz'schen Schrauben 22, 23, 23p in starrer Verbindung mit dem Verlängerungsnagel 32 getragen. Ist die vorgesehene maximale Länge a1 des Knochens 10 erreicht, werden -- wie bereits geschildert -- auch hier die Schrauben 22, 23, 23p durch Verriegelungsschrauben ersetzt.

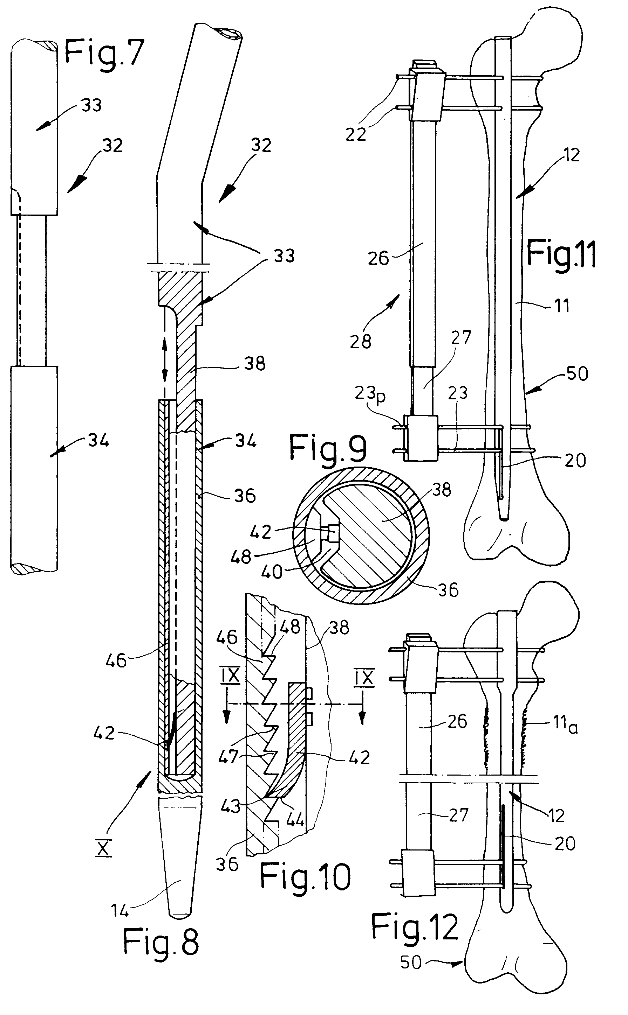

Beim Ausführungsbeispiel der Fig. 7, 8 ist der distale Nagelteil 34 in seinem oberen Bereich als Hülse 36 ausgestaltet, in die das stabartige Ende 38 des proximalen Nagelteils 33 einragt. Dieses Stabende 38 ist mit einer Längsnut 49 versehen, in welcher achsparallel eine Federzunge 42 festgelegt ist. Deren nach außen gekrümmtes Zungenende 43 wirkt mit einer in die Hülse 36 eingeformten inneren Zahnleiste 46 zusammen; die untere Zungenkante 44 kann an jeweils einer der nach oben gerichteten Schulter- oder Stufenflächen 48 der Zähne 47 als Anschlagkante anschlagen, wenn das Stabende 38 gegen die Hubrichtung x zu gleiten trachtet. Die Paarung Stufenfläche 48 / Anschlagfläche 44 wirkt somit als Rücklaufbremse.

Die Fig. 11, 12 sollen den Einsatz des Marknagels 12 bei einem Oberschenkelknochen 50 veranschaulichen.

Bei Einsatz des längenveränderlichen Marknagels 32 werden die erwähnten Schanz'schen Schrauben in ihm und in der Corticalis verankert. Der Marknagel 32 bleibt proximal sowie distal fixiert. Dabei wird die Belastung vom Fixateur 28 und den Schanz'schen Schrauben in starrer Verbindung mit dem Marknagel 32 getragen, wobei am Fixateur 28 keine Biegekräfte auftreten. Ist die gewünschte Knochenlänge erreicht, werden -- wie bereits erwähnt -- die Schanz'schen Schraube durch Verriegelungsschrauben ersetzt.

Durch die Verbindung der Schanz'schen Schrauben mit dem Marknagel 32 treten wesentlich geringere Biegekräfte auf den Verlängerungsapparat oder Fixateur 28 auf, und die Kräfte auf die Rundlöcher durch die Cortikalis müßten annähern gleich sein.

Die eigentliche Verbindung erfolgt durch manuelle, mechanische Krafteinflüsse über den Fixateur 28 außen, wobei die Kräfte auch elektrisch, hydraulisch oder pneumatisch erzeugt werden können.

## Patentansprüche

1. Vorrichtung zum Behandeln eines Knochens mit einem in diesen einsetzbaren Nagel, dessen Mittelachse Durchbrüche zur Aufnahme von Schrauben, Bolzen od.dgl. Stiftelementen queren, wobei an einem Ende des Nagels zumindest ein Rundloch, insbesondere ein Paar von Rundlöchern, als Durchbruch / Durchbrüche vorgesehen ist,
dadurch gekennzeichnet,
daß im anderen Endbereich des Nagels (12, 32) ein Längsschlitz (20) zur Aufnahme wenigstens einer darin gleitbaren Schraube (23,23b) eines Bolzens od.dgl. Stiftelement angeordnet ist und diese/s mit der/den Schraube/n (22) od.dgl. Stiftelement/en des anderen Nagelendes durch eine längenveränderbare Verstelleinrichtung (26) verbunden ist.

2. Vorrichtung nach Anspruch 1, dadurch gekennzeichnet, daß der Nagel (32) in sich längenveränderlich ausgebildet ist.

3. Vorrichtung nach Anspruch 1 oder 2, gekennzeichnet durch ein Paar von Rundlöchern (18) im proximalen Endbereich (16) des Nagels (12) und den Längsschlitz (20) in dessen distalem Bereich.

4. Vorrichtung nach einem der Ansprüche 1 bis 3, dadurch gekennzeichnet, daß dem Langloch oder Längsschlitz (20) zumindest eine Bohrung bzw. ein Rundloch (19) zugeordnet ist.

5. Vorrichtung nach Anspruch 4, dadurch gekennzeichnet, daß das Rundloch (19) in der vom Längsschlitz (20) bestimmten Geraden vorgesehen ist, oder daß im anderen Endbereich des Nagels (32) wenigstens ein Rundloch (18d) vorgesehen und der Nagel zwischen diesem und dem/den anderen Rundloch/Rundlöchern (18) in sich längenveränderlich ausgebildet ist, wobei in den Rundlöchern vorgesehene Schrauben (22 bzw. 23, 23b) durch eine Verstelleinrichtung (26) verbunden sind.

6. Vorrichtung nach Anspruch 1 bis 5, dadurch gekennzeichnet, daß die Verstelleinrichtung (26) in einem Abstand parallel zum Nagel (12, 32) verläuft.

7. Vorrichtung nach Anspruch 2 oder 6, dadurch gekennzeichnet, daß zwischen zwei gegeneinander bewegbaren Abschnitten (33, 34) des längenveränderlichen Nagels (32) eine Rücklaufbremse (44, 48) vorgesehen ist, wobei gegebenenfalls zwischen den Abschnitten (33,34) des längenveränderlichen Nagels (32) eine gegen die Einschubrichtung wirkende Rücklaufbremse vorgesehen ist.

8. Vorrichtung nach Anspruch 2, 6 oder 7, dadurch gekennzeichnet, daß zumindest ein Abschnitt (34) des Nagels (32) ein Hohlprofil und in diesem ein Stab (38) des anderen Abschnittes (33) kolbenartig verschiebbar ist, wobei gegebenenfalls zwischen den Abschnitten (33, 34) des längenveränderlichen Nagels (32) eine gegen die Einschubrichtung wirkende Rücklaufbremse vorgesehen ist.

9. Vorrichtung nach Anspruch 7 oder 8, dadurch gekennzeichnet, daß der Stab (38) oder die Innenseite des Hohlprofils (34) mit einer Zahnleiste (46) mit in Hubrichtung (x) weisenden Stufenflächen (48) versehen ist und der jeweilige Gleitpartner (34, 38) mit einem Rastorgan (42), welches mit einer Anschlagkante (44) auf eine der Stufenflächen aufsetzbar ist.

10. Vorrichtung nach einem der Ansprüche 7 bis 9, gekennzeichnet durch eine einends festgelegte Federzunge als Rastorgan (42), deren freies Ende die Anschlagkante (44) der Rücklaufbremse bildet.
